# EUROPEAN PATENT APPLICATION

(11) **EP 1 702 512 A1**
(43) Date of publication of application: **20.09.2006**
(21) Application number: 05005852.8
(22) Date of filing: 17.03.2005
(51) Int. Cl.: A01M 1/20

(54) **Device**

(71) Applicant: Reckitt Benckiser (UK) Limited, Slough, Berkshire SL1 3UH (GB)
(72) Inventor: McKechnie, Malcolm Tom, Dansom Lane, Hull, HU8 7DS (GB)
(74) Representative: Instone, Terry

(57) **Abstract**

A device for releasing to the air an active ingredient in vapour phase comprises a reservoir (1) which holds a liquid composition (8) in liquid contact with a volatilisation surface (2) wherein the composition comprises the active ingredient, a non-volatile liquid and a volatile liquid wherein the volatile liquid is soluble in the non-volatile liquid and the solution of volatile liquid in the non-volatile liquid has a lower surface tension than that of the non-volatile liquid itself.

## Description

The invention relates to a device for emitting a vapour, and to a method of delivering a vapour to an air. In particular, it relates to devices for releasing the vapour of active ingredients to the air, for instance to fragrance, deodorise, sanitise, treat allergens, deliver therapeutic agents (decongestants, mood enhancers), kill insects or repel insects. More specifically, it is concerned with devices which release the active ingredient passively and continuously over an extended time ranging from several minutes to several months.

Devices for the extended continuous release of vapour phase active ingredients into the air are known in the art.

One common type of device is a simple passive evaporation device. At the commencement of use a supply of the required chemical agent is exposed to the atmosphere, and starts to evaporate. Typically, in the traditional "pot pourri" type of air freshener, the active perfume ingredient, carried in solution in an oily liquid, is absorbed onto a porous solid (such as dried vegetable matter). The problem with this arrangement is that the release of active ingredient to the air is limited by diffusion through the porous solid carrier, and hence the rate of delivery of the active ingredient decreases over time and may be discarded while there is still active ingredient present within the solid carrier.

Attempts to overcome this problem have involved the use of devices with a reservoir holding a volatile liquid which is in liquid connection to a wick, in a manner similar to an oil lamp. However, these devices also suffer from reducing delivery rates during the lifetime of the device caused by diffusion gradients along the wick. In an attempt to overcome this, these devices have been provided with electrically powered heaters to increase the delivery rate of active ingredient to the air from the wick. In one device, described in Spanish Patent Application No. 9701388, the rate of evaporation is altered by varying the relative position of the wick and the heater.

It has now been found that by employing a particular blend of volatile and non-volatile liquids in a device provided with a volatilisation surface, problems of the prior art devices can be tackled without recourse to the need for an electrical power supply to drive the evaporation of the active ingredient.

Accordingly, a first aspect of the invention provides a device for releasing to the air an active ingredient in vapour phase, the device comprising a reservoir which holds a liquid composition in liquid contact with a volatilisation surface wherein the composition comprises the active ingredient, a non-volatile liquid and a volatile liquid wherein the volatile liquid is soluble in the non-volatile liquid and wherein the loss of the volatile liquid from the composition leads to an increase in the surface tension of the remaining composition.

It is thus preferred if the solution of the volatile liquid in the non-volatile liquid has a lower surface tension than that of the non-volatile liquid itself.

Without limiting the invention by any theory as to how it operates, it is thought that the improvements in evaporation of active ingredient are brought about through the Gibbs-Marangoni effect. It is thought that the composition, wets the volatilisation surface, and as a result the contact line between the composition and the volatilisation surface pulls a layer of liquid up to a certain height above the free surface of the composition.

As evaporation occurs from the thin layer of composition wetting the volatilisation surface, the more volatile liquid is lost more rapidly, leading to an increase in the surface tension of the thin layer. This increased surface tension drags more liquid up the volatilisation surface, leading to thickening of the upper edge of the wetting layer. Eventually, the thick layer becomes unstable and runs back down the volatilisation surface as droplets.

i.e. a balance between interfacial tension energy pulling the liquid up the emanating surface balanced against the gravitational pull to return the liquid to the reservoir.

It is thought that this recycling of the composition at volatilisation surface leads to continuous refreshing of the active ingredient in the composition at the volatilisation surface, to that the delivery rate of the active ingredient can be better maintained throughout the lifetime of the device.

The term "lifetime" in relation to the device of the present invention is used here to designate the period of time during which the device delivers an amount of active ingredient sufficient to be effective, i.e. which, for example, can be perceived in the case of perfumes, or can remain active as insect repellent, deodorizing or sanitizing agent, and the like.

The volatilisation surface can be the wall of the reservoir of the device, but it is preferably a separate surface which at a proximal end is immersed in the composition contained within the reservoir, and at a distal end protrudes upwardly from the free surface of the composition. The volatilisation is suitably substantially smooth and planar, or preferably it can be adapted to have a high surface area to volume ration by being curved or corrugated. Preferably any curves or corrugations have any radius of curvature of 0.5 mm or more, as smaller radii may interfere with the operation of the device. It is also preferred if the volatilisation surface is substantially smooth. If the volatilisation surface is corrugated, it is preferred if the axis of the corrugations is substantially vertical rather than horizontal.

The surface of the volatilisation surface must be wetted by the composition, by which is meant that the contact angle of the composition on the surface must be 90° or less, measured inside the liquid composition. Preferably the contact angle at 20°C is 45° or less, more preferably 10° or less, even more preferably 1° or less. This can be suitably measured visibly using a contact angle goniometer in a sealed chamber saturated with a vapour such that the composition is equilibrium with said vapour.

The reservoir may be fully open to the air but is preferably is provided with a covering means whereby the surface of the reservoir is substantially covered. The covering means is provided with an aperture whereby the volatilisation surface is in fluid connection with the ambient air. Preferably, the volatilisation surface projects through the aperture in the covering means. The covering means provides advantage of reducing the evaporation of the volatile liquid from the open surface of composition in the reservoir, so that the evaporation of the volatile liquid is used mainly for driving the operation of the device. It also prevents matter falling into the reservoir and inhibits spillage. The covering means may be removably attached to the reservoir or alternatively, the reservoir may be shaped so that it has a lower part for holding the liquid composition and an upper part shaped to form a covering means. For instance, the reservoir may be in the form of a sphere with a flattened base on which it can stand and an upper orifice at the top of the sphere, opposite the flattened base, through which the volatilisation surface extends, such that the covering means is formed by the upper part of the sphere. In another embodiment the covering means may be a lid with a hole in it removably or permanently attached to a reservoir in the form of a jar or dish.

Preferably, the device is also provided with a closure means separate from the covering means, whereby the reservoir, the composition and the volatilisation surface may sealed from vapour contact with the surrounding atmosphere in order to prevent evaporation from the device of the volatile liquid and the active ingredient, when the release of the active ingredient to the atmosphere is not needed, such as during storage or when the action of the device is not needed. The provision of the covering means has the advantage of prolonging the useful life of the composition and hence the functioning of the device. A suitable covering means is a lid, provided with a seal. A suitable seal is a screw fixture provided with an elastomeric washer. Suitably, the closure cap may be adapted to prevent opening of the closure cap by a child. Such tamper-proof closures are widely available and could be adapted for use in the present device.

The closure means may be removably attached or may preferably be fixed in place but provided with a permeable (by which is meant permeable to the volatile solvent and to the active ingredient) or open window, the window being provided with a shutter which is moveable from a closed position, sealing the window, and an open position, allowing fluid connection through the window between the ambient air and the volatilisation surface when the shutter is in the open position.

By volatile liquid, it is meant that the saturated vapour pressure of the liquid at 20°C is 3kPa or more. By involatile liquid, it is meant that the saturated vapour pressure (SVP) of the solvent at 20°C is less than 3kPa.

Preferably, the volatile liquid has an SVP at 20°C of 4kPa or more, preferably 5 kPa or more.

The volatile liquid must be soluble in the non-volatile liquid and suitably the surface tension of the solution is lower than the surface tension of A alone. By the statement that the volatile liquid is soluble in the non-volatile liquid, it is meant that the solubility of the volatile liquid in the non-volatile liquid is at least 0.2% by weight of non-volatile liquid. Preferably the volatile liquid has a solubility of at least 0.5% by weight of non-volatile liquid, more preferably at least 1% by weight of A. There is no upper limit on the solubility of volatile in non-volatile liquid by which it is meant that the two liquids may be completely miscible.

Where the term liquid is used, it is suitable for the invention that the liquid has a dynamic viscosity of 10000 mm²s⁻¹ or less at 25°C. Dynamic viscosities may be measured using a capillary viscometer. Preferably, liquids for the invention have a viscosity of 1000 mm²s⁻¹ or less at 25°C, more preferably 500 mm²s⁻¹ or less.

In a particularly preferred embodiment of the invention, the composition further comprises a precipitating component which is more soluble in the solution of volatile in non-volatile liquid than it is in the non-volatile liquid alone. The precipitating component may be volatile or non-volatile, but is preferably non-volatile. The precipitating component is preferably present in the composition at a level whereby particles (solid or liquid droplets) of the precipitating component are precipitated out of solution as the volatile liquid evaporates from the composition during use. In other words, the precipitating component is present at a level where it is soluble in the starting composition and where it is insoluble in the composition once a certain amount of the volatile liquid has been lost from the composition by evaporation during the use of the device. Such an arrangement provides the advantage of giving a visual indication to the user that the device must be changed or the composition replenished or replaced, As the composition becomes cloudy or opaque due to precipitation of the precipitating component when the volatile liquid has partly or entirely evaporated from the composition.

For example, if the non-volatile liquid is water and the volatile liquid is ethanol, the precipitating component may be a glycol ether that is only partially soluble in water but which is more soluble in the mix of water and ethanol. For example the precipitating component may be 1-butoxy-propan-2-ol. In another suitable embodiment, the precipitating component may be a perfume oil.

In other words, the active ingredient may act as the precipitating component, or a separate precipitating component may be included in compositions for use in the invention.

The volatile liquid and the non-volatile liquid may be chosen from a wide range of solvents, such as alkanes, alkenes, ethers, ketones, aldehydes, ehters, alcohols or mixtures thereof.

Preferred for the volatile liquid are ethanol and/or isopropyl alcohol.

Preferred for the non-volatile liquid is water or a glycol ether.

If the non-volatile liquid is a glycol ether, the glycol ether is suitably a (C1-4 alkyl)glycol(C1-4 alkyl) ether, di (C1-4 alkyl) glycol (C1-4 alkyl)ether,(C1-4 alkyl)glycol di (C1-4 alkyl)ether, di (C1-4 alkyl)glycol di (C1-4 alkyl)ether or tri (C1-4 alkyl) glycol (C1-4 alkyl) ether. The alkyl groups may have 1, 2, 3 or 4 carbon atoms and may be, for example, methyl, ethyl, n-propyl, i-propyl, n-butyl, i-butyl, s-butyl or t-butyl groups.

For example, the non-volatile liquid may be 1-butoxypropan-2-ol sold under then trade mark Dowanol PnB, propylene glycol monomethyl ether sold under the trade mark Dowanol PM, tripropylene glycol methyl ether sold under the trade mark Dowanol TPM, dipropylene glycol methyl ether sold under the trade mark Dowanol DPM, dipropylene glycol dimethyl ether sold under the trade mark Proglyde DMM, dipropylene glycol n-propyl ether sold under the trade mark Dowanol DPnP, dipropylene glycol mono n-butyl ether sold under the trade mark Dowanol DPnB, tripropylene glycol mono n-butyl ether sold under the trade mark Dowanol TPnB or propylene glycol n-butyl ether, or ethoxy propoxy propanol (EDP)

Suitably, the liquid composition of the present invention comprises 10% or more by weight of the volatile liquid , preferably 15% or more, more preferably 20% or more, even more preferably 25% or more. Preferably the composition comprises less than 40% by weight of volatile liquid.

The composition may consist essentially of the volatile liquid, the involatile liquid and the active ingredient. Suitably, other ingredients may be included which do not interfere with the operation of the device. In particular, a precipitating component which is more soluble in the solution of volatile in non-volatile liquid than it is in the non-volatile liquid alone as described above, may be included in the composition.

The level of involatile liquid is suitably 40% or more, preferably 50% or more, more preferably 60% or more by weight of the composition.

The surface tension of the involatile liquid is suitably 40 mN/m or more, preferably 55 mN/m or more, more preferably 70 mN/m or more.

The surface tension of the volatile liquid is suitably 30 or less, preferably 25 or less.

The solution of volatile in non-volatile liquid suitably has a surface tension 80% or less than that of the non-volatile liquid alone, preferably 65% or less, more preferably 50% or less.

The invention is concerned with the release of an active ingredient in the vapour phase into the atmosphere. Suitable active ingredients include fragrance, deodorant, sanitizing agent, insect repellent, insecticide, allergen treater or therapeutic agent and mixtures thereof.

Another aspect of the invention is concerned with the use of a composition which shows the Gibbs-Marangoni effect to deliver an active ingredient to the atmosphere from a volatilisation surface.

The invention also provides a method for delivering an active ingredient to the atmosphere from a volatilisation surface comprising the steps of:
a) preparing a liquid composition comprising the active ingredient as described above which demonstrates the Gibbs-Marangoni effect,
b) placing the liquid composition in a container in liquid contact with a volatilisation surface and in vapour contact with the atmosphere.

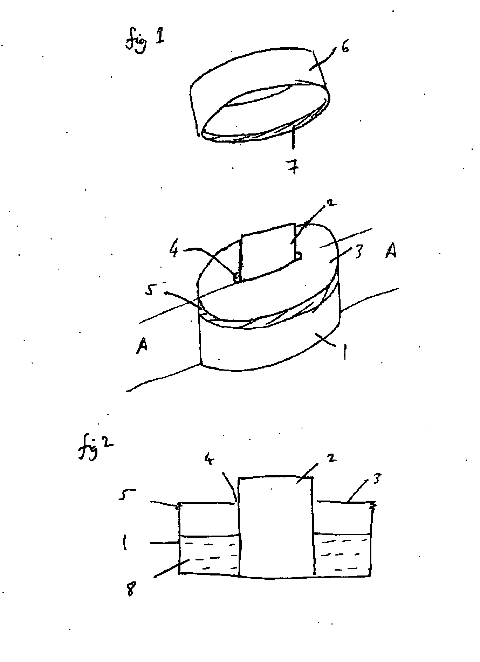

Figure 1 shows one embodiment of the invention in perspective. The closure means, a cylindrical cover (6), is shown separately in figure 1, has been removed from the device of figure 1, such that the volatilization surface of the device, the plate (2) is in vapour contact with the ambient atmosphere. Figure 2 shows a vertical cross-section through the plane A-A as defined in figure 1. The device has a cylindrical reservoir (1) furnished with a lid (3) as covering means. The lid has an aperture (4). The volatilisation surface (2) is attached to the base of the reservoir (1) and forms a vertical plane projecting out through the aperture (4). The upper circumference of the reservoir has a screw thread (5) to enable engagement with the cover (6), which is also fitted with a thread (7).

The reservoir (1) holds a liquid composition (8) which is in contact with the plate (2). The liquid composition is initially 60% water, 4% perfume and 36% ethanol.

Removal of the cover (5) leads to the composition rising up the surface of the plate (2) by the Gibbs-Marangoni effect, exposing a circulating liquid surface to the surrounding atmosphere whereby the vaporisation of the perfume is facilitated and a visual indicator of the functioning of the device is provided.

## Claims

1. A device for releasing to the air an active ingredient in vapour phase, the device comprising a reservoir which holds a liquid composition in liquid contact with a volatilisation surface wherein the composition comprises the active ingredient, a non-volatile liquid and a volatile liquid wherein the volatile liquid is soluble in the non-volatile liquid and wherein the loss of the volatile liquid from the composition leads to an increase in the surface tension of the remaining composition.

2. A device according to claim 1 wherein the active ingredient is a selected from the group consisting of fragrance, deodorant, sanitizing agent, insect repellent, insecticide , allergen treater, therapeutic agent and mixtures thereof.

3. A device according to any preceding claim wherein the non-volatile liquid is water.

4. A device according to any preceding claim wherein the volatile liquid is ethanol.
